# EUROPEAN PATENT SPECIFICATION

(11) **EP 1 444 261 B1**
(45) Date of publication and mention of the grant of the patent: **02.11.2005**
(21) Application number: 02787687.9
(22) Date of filing: 14.11.2002
(51) Int. Cl.: C07K 14/47

(54) **IMMUNOGENIC ALK (ANAPLASTIC LYMPHOMA KINASE) PEPTIDES**
IMMUNOGENE ALK (ANAPLASTIC LYMPHOMA KINASE) PEPTIDE
PEPTIDES ALK IMMUNOGENIQUES

(30) Priority: 15.11.2001 US 8377
(43) Date of publication of application: 11.08.2004
(73) Proprietor: ISTITUTO NAZIONALE PER LO STUDIO E LA CURA DEI TUMORI, I-20133 Milano (IT)
(72) Inventor: GAMBACORTI-PASSERINI, Carlo, I-20133 Milano (IT); PASSONI, Lorena, I-20133 Milano (IT)
(74) Representative: Banfi, Paolo
(86) International application number: PCT/EP2002/012764
(87) International publication number: WO 2003/042243

(56) References cited:
- WO-A-95/15331
- US-B1- 6 174 674
- DATABASE BIOSIS [Online] BIOSCIENCES INFORMATION SERVICE, PHILADELPHIA, PA, US; 16 November 2000 (2000-11-16) PASSONI LORENA ET AL: "In vitro induction of primary HLA class I-restricted immune response against ALK kinase." Database accession no. PREV200100312465 XP002243620 & BLOOD, vol. 96, no. 11 Part 1, 16 November 2000 (2000-11-16), page 338a 42nd Annual Meeting of the American Society of Hematology;San Francisco, California, USA; December 01-05, 2000 ISSN: 0006-4971
- PASSONI LORENA ET AL: "ALK as a novel lymphoma-associated tumor antigen: Identification of 2 HLA-A2.1-restricted CD8+ T-cell epitopes." BLOOD, vol. 99, no. 6, 15 March 2002 (2002-03-15), pages 2100-2106, XP002243621 March 15, 2002 ISSN: 0006-4971
- GAMBACORTI-PASSERINI C ET AL: "Mapping of HLA class I binding motifs in forty-four fusion proteins involved in human cancers." CLINICAL CANCER RESEARCH, vol. 3, no. 5, May 1997 (1997-05), pages 675-683, XP001149115 ISSN: 1078-0432
- PULFORD KAREN ET AL: "Immune response to the ALK oncogenic tyrosine kinase in patients with anaplastic large-cell lymphoma." BLOOD, vol. 96, no. 4, 15 August 2000 (2000-08-15), pages 1605-1607, XP002243622 ISSN: 0006-4971

## Description

### FIELD OF THE INVENTION

The present invention relates to an immunogenic peptide derived from Anaplastic Lymphoma Kinase (ALK) that can be used in the immunotherapy of tumors. More specifically, the invention provides a cytotoxic T-lymphocyte (CTL)-epitope from ALK protein, *in vivo* and *in vitro* methods, using the ALK peptide of the invention, for activating ALK-specific CTLs and for stimulating an immune response against ALK positive tumors, and pharmaceutical compositions suitable for use in such methods.

### BACKGROUND OF THE INVENTION

The discovery of tumor-associated antigens (TAAs) demonstrated that tumor cells can be specifically recognized by the immune system raising the hypothesis that most if not all tumors express antigens that cytotoxic T-lymphocytes can potentially attack (Renkvist N *et al*., (2001) *Cancer Immunol Immunother*., 50: 3-15). The identification of immunogenic epitopes led to their use as targets to mediate the specific clearance of neoplastic cells by TAA targeting strategies including vaccination, such as but not limited to allele-specific peptide-based vaccination. (Bremers AJ *et al*., (2000) *Eur J Surg Oncol*., 26: 418-424; Kugler A. *et al*.,(2000) *Nat Med*., 6: 332-336; Nestle FO *et al*.,(1989) *Nat Med*., 4: 328-332).

A major issue in the development of efficient vaccination protocols is the identification of the appropriate and effective TAAs, able to induce an immune response that will affect tumor growth. The ideal target for immune destruction is a tumor specific protein that is not expressed in normal cells and is essential for the malignant phenotype (Gilboa E., (1999) *Immunity*, 11: 263-270).

ALK is a receptor tyrosine kinase identified for the first time by Morris *et al*. (Morris SW *et al*., (1994) *Science*, 263: 1281-1284) as part of the fusion protein NPM/ALK (oncogenic anaplastic lymphoma kinase fusion protein) in ALCL-derived cell lines. More than half ALCL (anaplastic large cell lymphomas) cases possess a t(2;5) chromosomal translocation that leads to the expression of the NPM/ALK fusion protein. This fusion protein is composed of the N-terminal portion of the nuclear phosphoprotein Nucleophosmin (NPM) linked to the entire intracellular portion of ALK, which contains its kinase catalytic domain.(Morris SW *et al*., (1994) *Science*, 263: 1281-1284; Falini B *et al*., (1999) *Blood*, 93: 2697-2706). It has been extensively demonstrated that constitutively activated NPM/ALK is a potent oncogene having transforming and tumorigenic properties (Fujimoto J *et al*., (1996) *Proc. Natl. Acad. Sci*. U. S. A., 93: 4181-4186; Bischof D. *et al*., (1997) *Mol. Cell. Biol*., 17: 2312-2325; Kuefer *MU et al.,* Blood (1997) 90:2901-2910; Wellmann A. *et al*., (1997) *FASEB J*.,11: 965-972).

While NPM is a "housekeeping" gene that is ubiquitously expressed in normal cells, ALK expression is restricted in embryonic and adult mouse to the Central Nervous System (CNS) (Iwahara T *et al*., (1997) *Oncogene*, 14: 439-449; Morris S. *et al*., (1997) *Oncogene*, 14: 2175-2188). In humans, ALK was detected at low levels in some pericytes and scattered glial cells in specific areas of the CNS. ALK is not normally expressed in lymphohematopoietic tissues (Pulford K *et al*., (1997) *Blood*, 89:1394-1404). However, as a result of the t(2;5) chromosomal translocation, ALK is ectopically expressed at high levels in lymphoid cells and its constitutive tyrosine kinase activity leads to deregulated lymphocyte growth (Bischof D. *et al*., (1998) *Mol. Cell. Biol*., 18: 6951-6961).

The high level of NPM/ALK and its variants expression in lymphoma cells and their direct role in lymphomagenesis, combined with the fact that normal ALK is expressed at low levels in the immune privileged CNS, suggests that ALK could potentially be an ideal target for immunotherapy. Indeed, circulating antibodies recognizing NPM/ALK were recently detected in ALCL patient sera (Pulford K. *et al*., (2000) *Blood*, 96: 1605-1607).

The isolation of ALK peptides with HLA-A0201 binding motif and capable of inducing an *in vitro* immune response has been recently reported by Passoni *et al*. (2000) (Blood vol. 96 - abstract n. 1459) In particular, the decapeptide SLAMLDLLHV has been reported to stimulate CTL cytotoxicity against NPM/ALK-positive lymphoma cell lines.

### SUMMARY OF THE INVENTION

The present invention provides a peptide derived from anaplastic lymphoma kinase (ALK) which induce MHC class I restricted cytotoxic lymphocyte responses against tumor cells expressing the NPM/ALK fusion protein as a result of a t(2;5) translocation. The disclosed peptide is located in the kinase domain of the fusion protein, and represents and ALK epitope which is further characterized by spontaneous processing and presentation at the surface of tumor cells. Unlike point-mutated oncogenes, which are characterized by a restricted expression pattern that is limited to a small number of patients, translocated ALK is a widely expressed tumor-associated antigen characteristic of ALK-positive lymphomas (e.g., alkomas), neuroblastomas and ALK-expressing neoplasias. This feature makes ALK an attractive target for vaccination protocols.

In one embodiment, the invention provides a HLA-A*0201-binding ALK peptide (SEQ ID No. 2). The disclosed peptide is characterized by *in vivo* immunogenicity in HLA-A*0201 transgenic mice and the ability to stimulate MHC class I restricted ALK-specific CTLs *in vitro* using human peripheral blood lymphocytes obtained from healthy donors. The invention also provides a pharmaceutical composition comprising the ALK peptide of the invention in combination with at least one pharmaceutically acceptable excipient. In a particular embodiment, the invention provides a vaccine composition.

An alternative embodiment of the invention provides antibodies that selectively recognize the epitope represented by the disclosed ALK peptide. The ALK-specific antibodies encompassed by the invention include both monoclonal and polyclonal antibodies and active fragments thereof.

The invention further provides a method for inducing a cytotoxic response against tumor cells expressing an ALK antigen, which comprises contacting T lymphocytes with an ALK peptide under conditions suitable for CTL activation. In a particular embodiment the invention describes a method in which lymphocytes are contacted with antigen presenting cells comprising (e.g., carrying or presenting) the peptide of the invention bound to an HLA-A*0201 molecule. In an alternative embodiment the invention describes a method which comprises contacting lymphocytes directly with the ALK peptide of the invention.

In particular embodiments the invention relates to isolated antigen presenting cells, such as but not limited to HLA-A2.1 positive dendritic cells or autologus peripheral blood mononuclear cells, carrying the ALK peptide of the invention, as well as to isolated T lymphocytes which are capable of selectively binding a complex comprising an HLA class I molecule and the ALK peptide.

In yet another embodiment the invention provides the use of the ALK peptide of the invention for generating a pharmaceutical composition for treating tumors characterized by expression of an ALK antigen, such as an alkoma, a neuroblastoma or an ALK-expressing neoplasia. An alternative embodiment is the use of either antigen presenting cells carrying the ALK peptide or autologous T lymphocytes specific (e.g., capable of binding to or interacting with) for complexes of an HLA-A*0201 molecule associated with the ALK peptides for generating a pharmaceutical composition for treating tumors expressing an ALK antigen.

In yet other embodiments, the invention provides isolated nucleic acid molecules encoding the ALK peptide of the invention, chimeric genes comprising an isolated nucleic acid molecule of the invention under the control of a heterologous promoter, and expression vectors comprising a chimeric gene of the invention. These embodiments of the invention find utility in establishing a host vector system for the production of the ALK peptide of the invention, and for the isolation of host cells and cell lines comprising a chimeric gene of the invention.

### BRIEF DESCRIPTION OF THE DRAWINGS

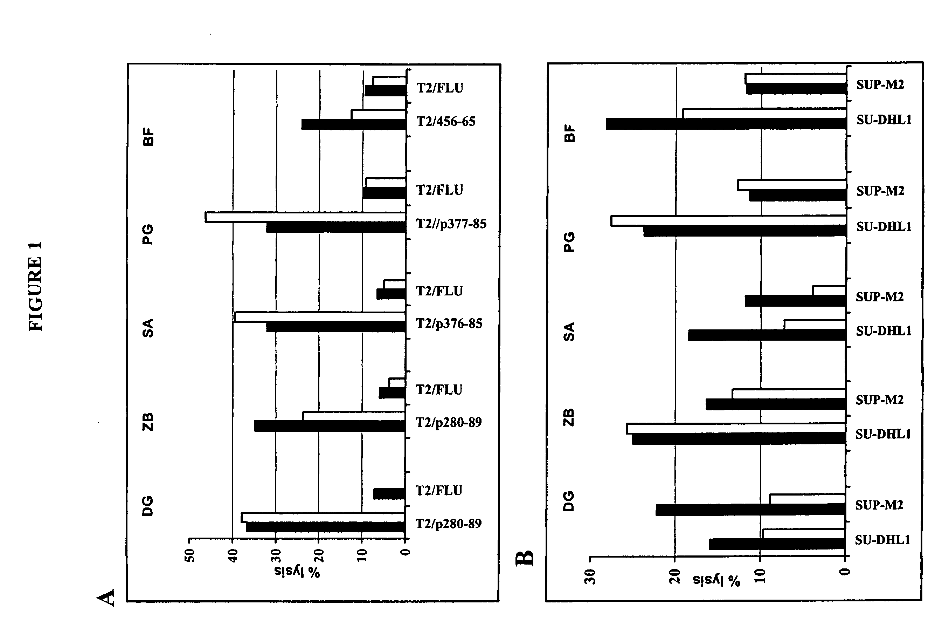

**Figures 1A and 1B. Induction of anti-ALK specific effectors in healthy donor Peripheral Blood Lymphocytes (PBLs)**. PBLs obtained from five donors (DG, ZB, SA, PG and BF) were activated with autologous peptide-pulsed DCs and restimulated weekly with peptide-loaded autologous monocytes. The resulting T-cell bulk cultures were tested for antigen specificity starting at the third round of stimulation. Cytotoxic activity was determined in a standard ⁵¹Cr-relese assay at an E:T ratio of 50:1 (■) and 25:1 (o) using as targets either T2 cells pulsed with 5 µM of cognate peptide or irrelevant influenza matrix-derived peptide (FLU) (Fig. 1A) or NPM/ALK positive lymphoma cell lines SU-DHL1 and SUP-M2 (Fig. 1B). Results of one representative experiment out of two performed are shown.

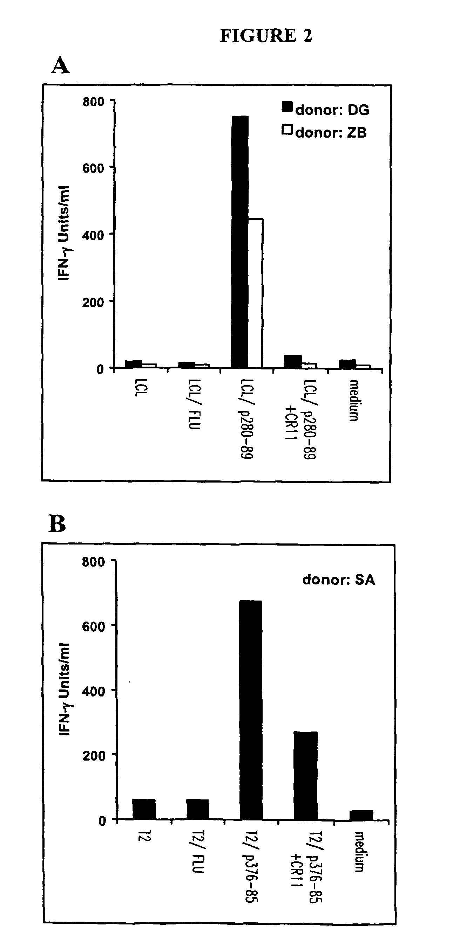

**Figures 2A and 2B. IFN-γ release by** ***in vitro*****-induced anti-ALK CTL lines**. Autologous EBV-transformed B cells (LCL) (Fig. 2A) and T2 cells (Fig. 2B) were pulsed with 5 µM p280-89 and p376-85 peptides, respectively as well as influenza matrix peptide (FLU: irrelevant peptide) and were then used as stimulator in an IFN-γ release assay. Preincubation with 5 µg/ml anti-HLA-A2 mAb CR11.351 inhibits IFN-γ secretion. Results of one representative experiment of at least three performed are shown.

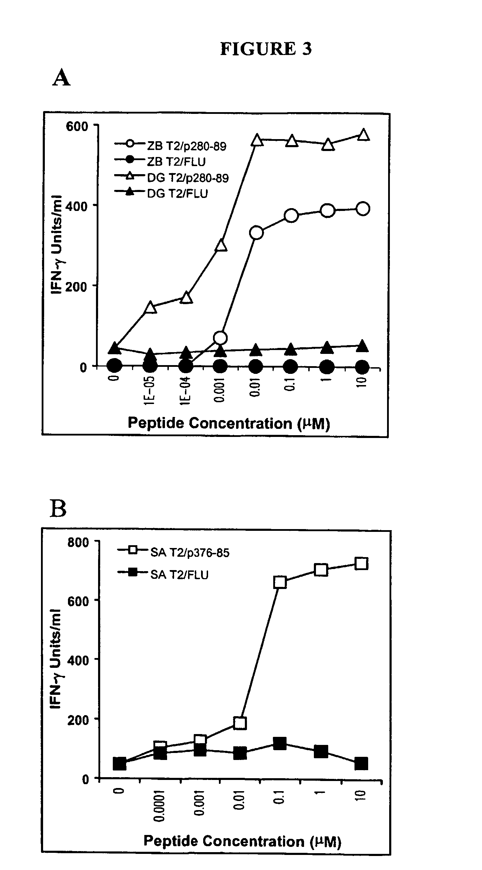

**Figures 3A and 3B. Peptide sensitivity of** ***in vitro*****-induced ALK-specific CTLs**. T2 cells were incubated with titrated amounts of p280-89 (Fig. 3A) and p376-85 (Fig. 3B) peptides as well as influenza matrix peptide (FLU) and were then used as stimulator in an IFN-γ release assay using CTL lines prepared from donors ZB and DG and SA. Results of one representative experiment out of two performed are shown.

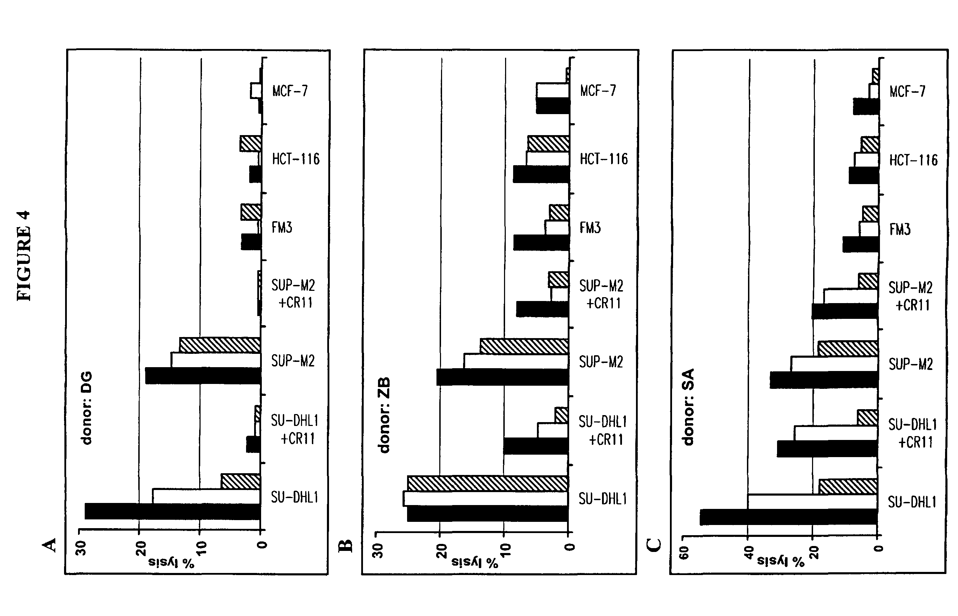

**Figures 4A - 4C. Specific lysis of ALCL cell lines endogenously expressing NPM/ALK**. p280-89-specific CTL lines generated from donors DG (Fig. 4A) and ZB (Fig. 4B), and p376-85-specific CTL lines generated from donor SA (Fig. 4C) efficiently recognize and lyse HLA-matched ALCL lymphoma cell lines (SU-DHL1 and SUP-M2) endogenously expressing NPM/ALK. Lysis was inhibited in the presence of anti-HLA-A2 mAb CR11.351 and no activity was detected against HLA-A2.1+ cell lines FM3 (melanoma), HCT-116 (colon carcinoma) and MCF-7 (breast carcinoma). Cytotoxic activity was determined in a standard ⁵¹Cr-relese assay at an E:T ratio of 50:1 (■), 25:1 (□), 12:1( 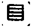 ). Results of one representative experiment of at least three performed are shown.

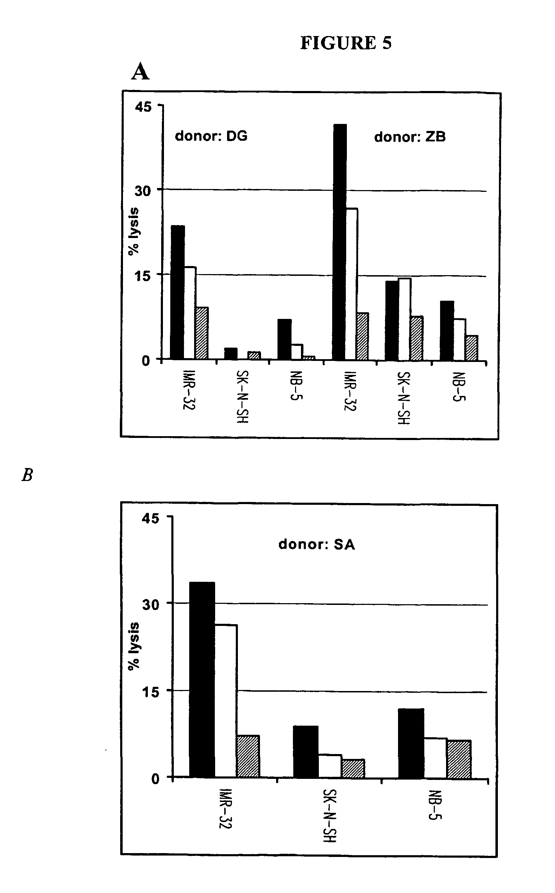

**Figures 5A and 5B. Lysis of neuroblastoma cells expressing native ALK.** Anti-p280-89 (Fig. 5A) and p375-86 (Fig. 5B) CTL lines recognize and kill neuroblastoma cells expressing native ALK in an HLA-restricted maner. IMR-32 (HLA-A2.1+/ALK+), SK-N-SH (HLA-A2.1-/ALK+) and NB-5 (HLA-A2.1+/ALK-) cell lines were used as targets in a standard ⁵¹Cr-relese assay using ALK peptide-specific ALK cell lines prepared from donors DG and ZB (p280-89-specific CTL lines) (Fig. 5A) and donor SA (p375-86-specific CTL line) (Fig. 5B). E:T ratio: 50:1 (■), 25:1 (□), 12:1( 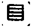 ). Results of one representative experiment of at least three performed are shown.

### DETAILED DESCRIPTION OF THE INVENTION

The invention is based on the identification of novel CTL epitopes within the NPM/ALK fusion protein. Specifically, a tumor-associated CTL epitope has been identified in the ALK tyrosine kinase domain, having the following properties: a) binding to HLA-A2.1 molecules, (b) immunogenicity *in vivo* in HLA-A*0201 transgenic mice, (c) *in vitro* stimulation of specific CTL responses using PBLs from healthy donors, (d) spontaneous processing and presentation at the surface of tumor cells.

Since the ALK protein is not expressed by normal cells, except at low levels in some CNS cells, the inventors investigated whether ALK kinase could be a target for antigen-specific cell-mediated immunotherapy. A panel of ALK-derived peptides were thus tested for their binding affinity to HLA-A*0201 molecules and then the binding peptides were assessed for their capacity to elicit a specific immune response mediated by Cytotoxic T-Lymphocytes (CTLs) both *in vivo,* in HLA-A*0201 transgenic mice, and *in vitro* in the peripheral blood lymphocytes (PBLs) from healthy donors.

Seven peptides able to bind HLA-A*0201 molecule and showing *in vivo* immunogenicity were thus identified. Of those, peptides p280-89 (SLAMLDLLHV, SEQ ID NO: 1), p376-85 (GVLLWEIFSL, SEQ ID NO: 2) p281-89 (LAMLDLLHV, SEQ ID NO: 3), p282-90 (AMLDLLHVA, SEQ ID NO: 4), and p377-85 (VLLWEIFSL, SEQ ID NO: 5) laid within the kinase domain, p456-65 (ALPIEYGPLV, SEQ ID NO: 6) was downstream of the kinase domain and p621-629 (SLTANMKEV, SEQ ID NO: 7) was in the C-terminal portion of the protein.

Two HLA-A*0201 restricted CTL epitopes, p280-89 (SLAMLDLLHV - SEQ ID NO: 1) and p375-86 (GVLLWEIFSL - SEQ ID NO: 2), were found to induce peptide-specific CTL lines able to specifically release IFN-γ upon stimulation with ALK peptide-pulsed autologous EBV-transformed B cells (LCLs) or T2 cells. Anti-ALK CTLs lysed HLA-matched ALCL and neuroblastoma cell lines endogenously expressing ALK proteins. CTL activity was inhibited by anti-HLA-A2 mAb CR11.351, consistent with a class I-restricted mechanism of cytotoxicity.

The results of the experiments where the ALK-peptides have been tested for their immunogenicity, confirm the existence of an efficient anti-ALK precursor population present within the T-cell repertoire of healthy donors. Considering the relatively low starting number of PBLs (36x10⁶) used to start each single sensitisation experiment, such precursors are probably present at high frequency. Given the known difficulty in generating immune responses *in vitro*, it is remarkable that the screening of only three healthy donors for each peptide induced a specific immune response in at least one donor, suggesting the strength of ALK in being immunogenic.

The anti-ALK specific CTL repertoire is unlikely to react against normal CNS cells, because the CNS is an immunologically privileged site where, under physiological conditions, antigens are not exposed to the immune system. More importantly, the scarce level of expression in normal CNS is most likely too low to ensure CTL recognition as shown with human MAGE-specific and murine p53-specific CTLs. In contrast, the high level of expression of NPM/ALK, driven by the strong NPM promoter, results in a substantial amount of NPM/ALK processing by the proteosome, thereby leading to a significant expression of ALK-derived epitopes at the cell surface. This renders the immune response against ALK selective for malignant cells.

The down-regulation of tumor antigen expression represents a problem for the development of immunotherapies, because of the possible immunoselection of non-expressing tumor clones. Since ALK is required for neoplastic transformation of lymphoid cells, tumor escape phenomena by ALK antigen loss variants are unlikely to occur.

Translocated ALK is expressed in approximately 83% and 31% of ALCL children and adult respectively (Drexler HG. *et al*., Leukemia. 2000; 14: 1533-1559), indicating that ALK is a stable lymphoma-specific hallmark. Recently, ALK-positive lymphoma or "Alkoma" has been recognized as a distinct tumor entity (Benharroch D *et al*., Blood 1998; 91: 2076-2084). ALK broad expression is not restricted to one or a few patients, as in the case of point-mutated oncogenes. This feature makes its use in vaccination protocols more easily and widely applicable.

Furthermore, ALK specific CTLs have been demonstrated to efficiently kill ALK+ neuroblastoma tumor lines. The killing of neuroblastoma cells by a CTL response specific for ALK epitopes reveals the potential of ALK as a shared tumor antigen for multiple malignancies.

In a first aspect, the invention provides the HLA-A*0201-restricted peptide SEQ ID NO: 2, which was found to strongly activate a cytotoxic response,

Because of its relatively short length, the peptide of the invention can be prepared in a wide variety of ways. For example, it can be synthesized in solution or on a solid support in accordance with conventional techniques. Various automatic synthesizers are commercially available and can be used in accordance with known protocols. For example, see Stewart and Young, (1984)*Solid Phase Peptide Synthesis*, 2d. ed., Pierce Chemical Co.; Tam et al., *J. Am. Chem. Soc*., (1983)105:6442; Merrifield, The Peptides, Gross and Meinenhofer eds. Academic Press, (1979)New York, pp. 1-284.

The peptide amino acid sequence according to the invention can be modified by deletion, substitution or addition of one or more residues, provided that the derivatives thus obtained have at least the same, preferably a higher immunogenic activity than the unmodified peptides. The latter can also be conjugated with lipids, glycosyl-residues or other peptides, to obtain improved properties such as higher affinity for the HLA molecule, higher immunogenicity or better bioavailability after administration. The peptides can also be conjugated with different epitopes known to induce a T "helper" cellular response through the same or a different class of MHC molecules.

The invention also includes isolated nucleic acid molecules encoding the HLA-A*0201-binding ALK peptide of SEQ ID NO 2. The choice of a specific nucleotide sequence coding for the ALK peptide will depend on the selected expression system and can be varied due to degeneracy of the genetic code. The invention also embraces an expression vector in which the pertinent nucleic acid sequence is operably linked to a heterologous promoter, as well as a host vector system for the production of a peptide which comprises said expression vector in a suitable host cell, and a cell line comprising the nucleic acid molecule encoding an ALK peptide.

Thus, the ALK peptide of the invention can be produced *in vitro* or *in vivo* as a consequence of the use of recombinant DNA technology to produce a nucleotide sequence which encodes an ALK peptide of interest. For example, a suitable coding sequence can be inserted into an expression vector, and the expression vector introduced into an appropriate host cell, by transformation or transfection, and the resulting host cell cultivated under conditions suitable for expression of the coding sequence and production of the encoded peptide. These procedures are generally known in the art, as described generally in Sambrook et al. *Molecular Cloning, A Laboratory Manual,* Cold Spring Harbor Press, Cold Spring Harbor, N.Y, (1982), and Ausuble et al., (ed.) *Current Protocols in Molecular Biology*, John Wiley and Sons, Inc., New York (1987). Thus, it is well within the skill of one skilled in the art to prepare and produce a nucleic acid sequence that encodes the ALK-peptide of the invention. Suitable nucleotide sequence can be back-translated using codons that encode the amino acids of any one of the ALK peptides disclosed herein that has been demonstrated to be immunogenic for human lymphocytes.

Alternatively, the coding sequence for peptides of the length contemplated herein can be synthesized by chemical techniques, for example, the phosphotriester method of Matteucci et al., (1981) *J. Am. Chem. Soc*., 103:3185. Chemical synthesis provides the opportunity to modify the coding sequence to include appropriate linkers designed to facilitate ligation into commonly available and well-known expression vectors. Numerous vectors and suitable host systems are widely available in the art. Generally speaking, for expression of the peptide, the coding sequence will be provided with operably linked stop and start codons, promoter and terminator regions and usually a replication system to provide an expression vector for a particular cellular host. For example, bacterial, yeast or mammalian host cells may be used in combination with appropriate vectors and control sequences.

According to a further aspect, the invention is directed to pharmaceutical compositions containing an effective amount of the peptide herein described. The pharmaceutical compositions for therapeutic treatment are intended for parenteral, oral or local administration. Preferably, the pharmaceutical compositions are administered parentally, e.g., intravenously, intramuscularly. Actual methods for preparing and parentally administering the pharmaceutical compositions of the invention will be known or apparent to one of skill in the art and are described in more detail in for example, Remington's Pharmaceutical Science, 17^{th} ed., Mack Publishing Company, Easton, PA (1985).

In a preferred embodiment, said compositions are in the form of vaccines, particularly suited for preventive vaccination of subjects with cancer susceptibility or for therapeutic vaccination of neoplastic patients. In addition to the active ingredients, the vaccine compositions will contain one or more pharmaceutically acceptable excipients.

"Effective amounts" means an amount sufficient to elicit an effective CTL response against a tumor cell. Such amount will depend on the route and type of administration, on the severity of the pathology to be treated and on the general conditions of the patient; suitable amounts will usually vary from 1 to 1000 µg, preferably from 100 to 300 µg divided in three or more administrations at 1 month intervals or more frequently. General procedures for the preparation and use of vaccines are known to those skilled in the art (see for example Paul, Fundamental Immunology, Raven Press, New York (1989) or Cryz, S. J., Immunotherapy and Vaccines, VCH Verlagsgesselshaft, 1991). Vaccines are usually prepared in the form of injectable suspensions or solutions, but they can also be used in the form of solid or liposome preparations. The immunogenic ingredients are preferably mixed with one or more pharmaceutically acceptable excipients, such as emulsifiers, buffering agents or adjuvants enhancing the efficacy of the vaccine. The vaccine can be administered according to a single or multiple dosage scheme. In case of multiple dosage, 1 to 10 separated doses are provided, each containing an antigen amount ranging from 1 to 1000 µg, followed by booster doses at different time intervals necessary to maintain or strengthen the immune response and, if necessary, a further dose after several months. In each case, the treatment regimen will depend on the response of the patient and on tumor progression.

The compositions and methods of the invention may also be employed for *ex vivo* therapy. By *ex vivo* therapy is meant that therapeutic or immunogenic manipulations are performed outside of the body. For example, lymphocytes or antigen presenting cells may be removed from a patient and treated with high doses of the subject peptides, providing a stimulatory concentration of peptide in the cell culture medium far in excess of levels that could be accomplished or tolerated by the patient if the peptide were to be administered *in vivo*. Following treatment the cell population (e.g., cytotoxic T lymphocytes, bulk lymphocyte populations or antigen presenting cells) can be returned to the host. Alternatively, a patients cells may be exposed to vectors that carry a nucleic acid sequence that encodes the ALK peptide of the invention. Once transfected, the cells may either be propagated *in vitro* until a desired cell density is achieved or returned directly to the patient for in vivo expansion.

More specifically, the invention provides a method for inducing a cytotoxic response against tumor cells expressing one or more ALK epitopes, which comprises contacting T lymphocytes with the peptide of the invention in conditions suitable for the activation of cytotoxic T cells. Suitable conditions for achieving the desired cytotoxic effects comprise direct exposure of T lymphocytes to the peptide in culture or to antigen presenting cells (APC) expressing HLA class I molecules previously loaded with (e.g., carrying) the peptide. Suitable APCs are peripheral blood autologous mononuclear cells (PBMC), dendritic cells, macrophages or activated B cells. An APC culture is added with the peptide for a time sufficient for binding and then with a cell population containing T lymphocytes which are thus activated to proliferate and to mediate cytotoxicity.

Lymphocytes can be taken from the patient under treatment and, after activation, re-injected into the same patient. Peptide/APC binding can be increased by previously "stripping" the histocompatibility molecules present on APCs. Optionally patient APCs can be genetically engineered to express the HLA-A*0201 allele. For example, the allele HLA-A2.1 is accessible in GenBank No.: M84379, protein sequence No.: PID g403144.

Furthermore, the culture medium can contain one or more cytokines to increase the activation of CD8+ precursors. Prior to their reintroduction into the patient, lymphocytes can be purified for example through an affinity column derivatized with a specific ligand. For example, T lymphocytes can be selectively enriched by employing an affinity column derivitized with a reagent, such as an antibody or active fragment thereof, that binds to CD3, CD8 or CD4.

A further object of the invention are antigen presenting cells (APCs), preferably dendritic cells, carrying (presenting) the peptide of the invention. As used herein the term carrying refers to the molecular complex that results from a binding interaction between an ALK peptide of the invention and an HLA class I molecule. The APCs can be genetically modified, for example by transfection with suitable viral or retroviral vectors, so as to confer the requisite, intracellularly process and molecular species required to express the relevant ALK peptide in a MHC Class I restricted manner on the cell surface. Alternatively, the APCs can be loaded with the peptide in culture.

The invention also provides a cytotoxic T cell specifically recognizing a complex formed by an HLA class I molecule, preferably HLA-A*0201, and the peptide SEQ ID NO 2. The cytotoxic T-lymphocyteline can be obtained by selection, starting from a lymphocyte pool, of cells capable of activating upon exposure to tumor cells containing ALK antigenic determinants.

In a further embodiment, the invention provides a method for treating a subject having a disorder characterized by expression of ALK antigens, which comprises administering to the subject an effective amount of the ALK peptide SEQ ID NO 2, or an amount of APCs carrying such an ALK peptide, or of autologous T lymphocytes specific for complexes of an HLA-A*0201 molecule and an ALK peptide of the invention. Disorders that can benefit from such a treatment include, but are not limited to, ALK-positive lymphomas and neuroblastoma.

A further embodiment of the invention relates to antibodies, fragments or derivatives thereof, recognizing and binding the peptide of the invention. Methods for producing antibodies to specific peptides are well known in the art (Kohler and Milstein, Nature 256 (1975), 494, or J.G.R. Hurrel, Monoclonal Hybridoma Antibodies: Techniques and Applications, CRC Press Inc., Boco Raton, FL, 1982). The antibodies according to the invention include monoclonal and polyclonal antibodies as well as their F(ab')2, Fab, Fv or scFv fragments.

### EXPERIMENTAL

### EXAMPLE 1: HLA-A*A0201 binding assays

To determine the binding ability of predicted peptides to HLA-A*0201 molecules, an *in vitro* cellular binding assay was performed using the TAP-deficient cell line T2. As used herein, the term TPA refers to "transporter associated with antigen presentation." T2 cells are HLA-A*0201 positive, TAP-negative human cells. T2 cells were incubated with 100 µM of peptide in serum-free RPMI 1640 supplemented with 5 µg/ml of human β₂m (Fluka, Buchs, Germany) for 18 hrs at 37°C. HLA-A*0201 expression was then measured by flow cytometry using the anti-HLA-A2.1 mAb BB7.2 followed by incubation with FITC-conjugated F(ab')₂ goat anti-mouse Ig (BioSource, Camarillo, CA). The ability of the ALK-derived peptides to bind to HLA-A*0201 molecules was assessed by measuring the binding affinity, expressed as Fluorescence Index (FI) defined as a ratio (median channel of fluorescence) between the sample and a control containing an irrelevant peptide (PML/RARα: VASGAGEAA). An increase over the control of at least 65% (FI > 2) was arbitrarily chosen as the cutoff.

To assess the HLA-A*0201/peptide complex stability, T2 cells (10⁶/ml) were incubated overnight with 100 µM of each peptide in serum free RPMI 1640 supplemented with 100 ng/ml of human β ₂m at 37°C. Cells were then washed four times to remove free peptides, incubated for 1 hr with 10 µg/ml of Brefeldin A (Sigma-Aldrich, France) in order to block cell surface expression of newly synthesized HLA-A*0201 molecules, washed and incubated at 37°C for 0, 2, 4, 6, or 8 hours. Subsequently, cells were stained with anti-HLA-A2.1 mAb BB7.2. For each time point, peptide induced HLA-A*0201 expression was calculated as: mean fluorescence value of peptide incubated T2 cells/mean fluorescence value T2 cells in the absence of the peptide. Dissociation Complex₅₀ (DC₅₀) was defined as the time required for the loss of 50% of the HLA-A*0201/peptide complexes stabilized at t=0.

The peptide binding ability and the HLA-A*0201/peptide complex stability are reported in the Table 1.

### EXAMPLE 2 - In vivo immunogenicity of ALK-derived peptides in HHD mice

### Animals

The HHD (Human Human D^{b}) transgenic mice have been previously described. Pascolo, S. *et al*., (1997) *J. Exp. Med*., 185:2043; Firat, *H., et al*., (1999) *Eur. J. Immunol*., 29: 3112). HHD mice are deficient for the β ₂-microglobulin (β ₂m) gene and for mouse MHC class I H-2D^{b} molecules and are transgenic for a chimeric HLA-A*0201/D^{b} molecule. More specifically, they are β ₂m-/-, D^{b}-/- double knockout and express a transgene encoding an HLA-A*0201 monochain composed of a chimeric heavy chain (α1 and α2 domains of human HLA-A*0201, α 3 and intra-cellular domains of D^{b}) linked by its N-terminus to the C-terminus of the human

β ₂-microglobulin (β ₂m) light chain by a 15 amino acid peptide. The chimeric molecule is the only class I molecule that is serologically detected on the surface of HHD cells.

Mice were housed in a temperature-controlled, light cycled room. All *in vivo* experiments were performed in accordance with local ethical guidelines.

### Procedure

To evaluate the *in vivo* immunogenic potential of ALK-derived high-affinity binding peptides, we immunized H-2D^{b-/-}, β2m^{-/-}, HLA-A*0201 monochain transgenic (HHD) mice. In these mice, the peripheral CD8+ T cell repertoire is educated, both at the thymic and peripheral levels, with transgenic HLA class I human molecules. Therefore, the HHD mouse is a useful animal model to assess the ability of individual peptides to induce HLA-A*0201-restricted CTL responses *in vivo* (Firat H. *et al*., (1999) Eur J Immunol. 29: 3112-3121; Minev B. *et al*., (2000) Proc Natl Acad Sci U S A. 97: 4796-4801).

HHD mice were injected subcutaneously at the base of the tail with 100 µg of peptide emulsified in Incomplete Freund's Adjuvant (IFA) in the presence of 140 µg of IA^{b} restricted HBV core antigen-derived T-helper epitope (p128-40; TPPAYRPPNAPIL). After 11 days, spleen cells from primed mice (5x10⁷ cells in 10ml) were restimulated *in vitro* with 10 µM of peptide. On day 6 of culture, cells were tested for specific cytotoxicity in a 4-hrs ⁵¹Cr-release assay using as targets RMA-S/HHD cells pulsed with 1µM peptide or alone.

As indicated in Table 1, immunization of the HHD mice with ALK-derived peptides induced peptide-specific CTL responses.

**TABLE 1**

| Affinity of ALK-derived Peptides for HLA-A*0201 Molecules and Immunogenicity in HHD Mice | | | | |
|---|---|---|---|---|
| PEPTIDE (*) | FI (**) | DC₅₀(hs) (***) | R/T HHD mice (****) | % lysis (*****) |
| p280-89 | 5.6 ± 0.92 | 4 | 3/6 | 63 - 49 - 18 |
| p376-85 | 2.8 ± 0.17 | 6 | 3/6 | 24 - 15 - 15 |
| p282-90 | 5.4 ± 1.78 | 4-6 | 3/6 | 80 - 56 - 50 |
| p377-85 | 3 ± 0.35 | 4-6 | 3/6 | 48 - 24 - 21 |
| p621-29 | 3.5 ± 0.16 | 4-6 | 2/6 | 63-53 |
| p281-89 | 3.5 ± 0.65 | 2 | 2/6 | 42 - 34 |
| p456-65 | 4.2 ± 0.25 | 2-4 | 2/6 | 67 - 15 |

| | | | | |
|---|---|---|---|---|
| * Position is referred to NPM/ALK sequence. | | | | |
| ** Ratio (median channel of fluorescence) between the sample and a control containing an irrelevant peptide. Results are expressed as the mean value of 4 experiments ± standard error mean. | | | | |
| *** DC50: Half-life of disassociation of HLA-A*02.01/peptide complexes. | | | | |
| **** R/T, responder versus tested mice. Mice were considered as responders when the specific lysis of peptide-loaded RMA-S/HDD target cells was at least 15% above the lysis of RMA-S/HDD. | | | | |
| ***** Percent lysis of peptide-loaded RMA-S/HDD cells at an E:T ratio of 60:1. Values correspond to the maximal lysis observed for each responder mouse. | | | | |

### EXAMPLE 3 - Stimulation of ALK-specific CTLs derived from healthy donor PBLs

Blood was collected from HLA-A*0201 normal volunteer donors. PBMC were isolated from whole blood by Ficoll/Hypaque (Pharmacia, Uppsala, Sweden) density gradient centrifugation. Dendritic cells (DCs) were generated according to the protocol of Sallusto and Lanzavecchia (Sallusto F. *et al*., (1994) *J Exp Med*.179: 1109-1118). Adherent monocyte-enriched peripheral blood mononuclear cells (PBMCs) were cultured in RPMI 1640 medium supplemented with 10% FCS,

20 ng/ml recombinant human interleukin (IL)-4 (R&D Systems, Minneapolis, MN) and 800 U/ml recombinant human granulocyte-macrophage colony-stimulating factor (GM-CSF) (Sandoz, Basel, Switzerland). On day 5 of culture, 10ng/ml recombinant human tumor necrosis factor (TNF)-α (Knoll, Ludwigshafen, Germany) was added to the medium. After 40 hrs, the surface phenotype of monocyte-derived DCs was determined by single-color flow cytometry using the following mAbs: fluorescein-coniugated anti-CD1a (Valter Occhiena, Torino, Italy), anti-CD86 (CALTAG, Burlingame, CA), anti-CD83 (CALTAG, Burlingame, CA), anti-CD14 (Becton Dickinson) and rhodamine-conjugated anti-CD80 (Becton Dickinson). DCs were used to prime autologous PBL as follows. DCs were pulsed with 10 µM peptide for 2 hrs at 37°C, irradiated at 30 Gy and added to autologous PBLs at a ratio variable from 3:1 to 6:1 (PBL:DC). PBLs (36x10⁶ total) were cultured at 1.5x10⁶ cells/2ml of Culture Medium (CM: 50% RPMI 1640 / 50% X-VIVO (BioWhittaker, Walkkersville, MA)) supplemented with 10% of heat-inactivated autologous plasma and 10 ng/ml IL-7 (R&D Systems, Minneapolis, MN) in the presence of autologous peptide-loaded DCs. At day 10-12 of culture, lymphocytes were restimulated with irradiated (30Gy) autologous peptide-loaded monocytes in CM supplemented with 10% of heat-inactivated autologous plasma. After 24-48 hrs 10 IU/ml IL-2 (Chiron Co., Emeryville, CA) and 10 ng/ml IL-7 was added to the CM. Lymphocytes were then restimulated weekly in the same way. Starting from the forth round of stimulation, the specificity of the resulting T-cell lines was evaluated weekly in a cytolytic assay. When any specific activity was detected, CD8+ T-cells were isolated from the bulk culture by CD4+ cells negative depletion using mAb-coated beads (Dynal Dynabeads, Oxoid, Oslo, Norway) and the CTL lines were maintained in culture in RPMI 1640 containing 10% human AB serum supplemented with 50 U/ml IL-2 in the presence of irradiated (70Gy) PBMCs as feeder.

For cytokine release assay, 10⁵ target cells (EBV-transformed B cells and TAP-/- T2 cells) and 10⁵ lymphocytes were incubated overnight in roundbottomed microtiter plates in 250 µl/well total volume of RPMI 1640 supplemented with 10% FCS. Plates were then centrifuged and 200 µl aliquot of supernatant was removed from each well. IFN-γ level was determined by ELISA (MABTECH, Nacka, Sweden).

### Results

To prime ALK-specific lymphocytes, peptide-pulsed autologous dendritic cells (DCs) derived from peripheral blood mononuclear cells (PBMCs) were used after their *in vitro* maturation was confirmed by flow cytometric analysis of DC-specific cell surface markers. After a 3-4 week cycle of restimulation T lymphocyte specificity for ALK peptides was evaluated by determining antigen-restricted lysis of peptide loaded T2 cells (Fig. 1A) and NPM/ALK-positive lymphoma cell lines (Fig. 1B).

At least one out of the three donors tested were found to be responders to stimulation with peptides p280-89 (SEQ ID NO: 1), p376-85 (SEQ ID NO: 2), p377-85 (SEQ ID NO: 5) and p456-65 (SEQ ID NO: 6) (donors DG, ZB, SA, PG, and BF) (Figures 1A and 1B). No specific CD8 reactivity was detected in the cell lines generated from donor PBLs stimulated with peptides p281-89 (SEQ ID NO: 3), p282-90 (SEQ ID NO: 4), and p621-29 (SEQ ID NO: 7) (data not shown).

Attempts at generating anti-ALK CTLs from responding donors led to the establishment of two cell lines specific for peptide p280-89 (donors DG and ZB) and one line specific for peptide p376-85 (donor SA). Flow cytometric analysis demonstrated that more than 99% of the cells were CD3+, CD8+, CD4- (data not shown).

Antigen specificity of anti-ALK specific CTLs was investigated. Autologous EBV-transformed B cells (LCL) or T2 cells were pre-pulsed with the indicated peptide and used as targets under different conditions (Figure 2). CTLs from the two donors responding to ALK peptide p280-89, released IFN-γ (DG: 750 IU/ml; ZB: 445 IU/ml) when incubated with p280-89 loaded LCL. The cytokine secretion was completely blocked in the presence of anti-HLA-A2 mAb CR11.351. No IFN-γ. production was observed when CTLs were incubated with autologous LCL alone, or with LCL cells loaded with irrelevant peptide (influenza matrix: FLU; Figure 2A). Similar quantities of IFN-γ (674 IU/ml) were released by CTLs generated from donor SA and incubated with p376-85 pulsed T2 cells. No, or limited quantities of, IFN-γ was detected after incubating CTLs in the presence of anti-HLA-A2 mAb CR11.351, alone or with FLU-pulsed T2 cells (Figure 2B).

A titration assay was performed to further assess the sensitivity and competence of CTLs in recognizing the antigen. T2 cells were pulsed with p280-89, p376-85 or FLU peptides at different concentrations, and then used as stimulators in a cytokine release assay. For p280-89 specific CTLs, maximal production of IFN-γ was reached with only 10 nM of peptide, indicating high affinity of recognition and antigen concentration-dependent IFN-γ production (Figure 3A). The CTL line from donor SA, specific for ALK peptide p376-85, exhibited a lower level of IFN-γ release (maximal release at 100 nM), indicating a lower efficiency for T cell stimulation (Figure 3B).

Taken together these results indicate that ALK peptides, p280-89 and p376-85, previously tested for their ability to be immunogenic in HHD mice, were also able to mobilize a specific CTL repertoire in PBLs from healthy donors. Moreover, dose-response data indicated a high and a moderate affinity of recognition for p280-89 and p376-85 generated CTLs respectively, providing further evidence that ALK kinase is immunogenic and not subject to tolerance mechanisms.

### EXAMPLE 4 - Lysis of tumor cell lines

Cytolytic activity was measured in a conventional 4-hrs ⁵¹Cr release assay. A 10-fold excess of unlabeled K562 cells was added to offset NK activity. Target cells and E:T ratio are indicated in each figure. Specific lysis was determined according to the following formula: % specific lysis = cpm (sample - spontaneous)/cpm (total - spontaneous) x 100. HLA-A*0201 blocking of T-cell activity (lysis and/or citokine release) was performed by preincubating target cells with the anti-HLA-A2 mAb CR11.351.

To determine if ALK-derived peptides, demonstrated to be immunogenic *in vivo* and *in vitro*, were also naturally processed on human tumor cells, we examined the ability of p280-89-specific and p376-85-specific CTLs to lyse HLA-A2.1+ ALCL lymphoma cell lines, SU-DHL1 and SUP-M2, expressing the chimeric protein NPM/ALK. ALK specific CTLs killed these tumor lines and their lytic activity was inhibited by the presence of anti-HLA-A2 mAb CR11.351. No lysis above background level was observed in HLA-A2.1+/ALK- FM3 (melanoma), HCT-116 (colon carcinoma), MCF-7 (breast carcinoma) human tumor lines expressing irrelevant antigens (Figure 4). Thus, it can be concluded that ALK peptide induced CTLs efficiently lyse lymphoma targets, in a HLA-A2 restricted fashion, by relevant antigen-specific recognition.

Native ALK tyrosine kinase expression has also been demonstrated in cell lines and tumors of neural origins such as neuroblastoma (Lamant L, *et al*.(2000) *Am. J. Pathol*., 156: 1711-1721). Because p280-89 and p376-85 peptides lie within the kinase domain, which is present in both native and translocated ALK, we used neuroblastoma cell lines as targets in cytotoxic experiments to further verify the natural processing of these two epitopes. Due to the limited expression of class I MHC molecules characterizing neuroblastomas that could interfere with the antigen recognition by CTLs, we treated tumor cells with INF-γ before their use as targets. p280-89-specific and p376-85-specific CTLs efficiently lysed HLA-A2.1+/ALK+ neuroblastoma cell line IMR-32. No lysis was detected either for HLA-A2.1-/ALK+ neuroblastoma cell lines SK-N-SH or for HLA-A2.1+/ALK- NB-5 cell line (Figure 5).

These results indicate that both p280-89 and p376-85 epitopes are efficiently processed and presented for CTL recognition at the cell surface of tumor cells and may therefore provide targets for antigen-specific immunotherapy.

## Claims

1. The HLA-A*0201-binding ALK peptide SEQ ID NO:2.

2. A monoclonal or polyclonal antibody, or an active fragment thereof, recognizing and binding the ALK peptide SEQ ID NO: 2.

3. A pharmaceutical composition comprising the ALK peptide according to claim 1 in combination with at least one pharmaceutically acceptable excipient.

4. The composition according to claim 3, in the form of a vaccine.

5. A method for inducing ex vivo a cytotoxic response against tumor cells expressing an ALK antigen, which comprises contacting T lymphocytes with the peptide according to claim 1 in suitable conditions for T lymphocyte activation.

6. The method according to claim 5, wherein the T lymphocytes are directly exposed to the peptide in culture.

7. The method according to claim 5, wherein the peptide is previously bound to the HLA-A*0201 molecule.

8. An isolated antigen presenting cell carrying the ALK peptide according to claim 1.

9. The antigen presenting cell according to claim 8, wherein the cell is a dendritic cell.

10. An isolated T lymphocyte which selectively binds a complex of an HLA-class I molecule and the ALK peptide SEQ ID NO:2.

11. The use of the ALK peptide SEQ ID NO:2, or an antigen presenting cell carrying such a peptide, or autologous T lymphocytes specific for complexes of an HLA-A*0201 molecule and the ALK peptide as above specified, for the preparation of a pharmaceutical composition for treating tumors expressing an ALK antigen.

12. The use according to claim 11, wherein said tumor is selected from the group consisting of: an ALK-positive lymphoma, a neuroblastoma and an ALK-expressing neoplasia.

13. An isolated nucleic acid encoding the HLA-A*0201-binding ALK peptide SEQ ID NO: 2.

14. A chimeric gene comprising the isolated nucleic acid of claim 13 under the control of a heterologous promoter.

15. An expression vector comprising the chimeric gene of claim 14.

16. A host vector system for the production of a peptide which comprises the expression vector of claim 15 in a suitable host cell.

17. A cell line comprising the chimeric gene of claim 14.

## Patentansprüche

1. Das HLA-A*0201-bindende ALK-Peptid SEQ ID No: 2.

2. Monoklonaler oder polyklonaler Antikörper, oder aktives Fragment davon, welcher das ALK-Peptid SEQ ID No: 2 erkennt und bindet.

3. Pharmazeutische Zusammensetzung, umfassend das ALK-Peptid nach Anspruch 1 in Kombination mit mindestens einem pharmazeutisch verträglichen Exzipienten.

4. Zusammensetzung nach Anspruch 3 in Form eines Impfstoffs.

5. Verfahren zur ex vivo-Induktion einer cytotoxischen Antwort gegen Tumorzellen, welche ein ALK-Antigen exprimieren, welches In-Kontakt-Bringen von T-Lymphocyten mit dem Peptid nach Anspruch 1 unter geeigneten Bedingungen für die T-Lymphocyten-Aktivierung umfasst.

6. Verfahren nach Anspruch 5, wobei die T-Lymphocyten gegen das Peptid in Kuftur direkt exponiert werden.

7. Verfahren nach Anspruch 5, wobei das Peptid zuvor an das HLA-A*0201-Molekül gebunden wird.

8. Eine ein isoliertes Antigen aufweisende Zelle, die das ALK-Peptid nach Anspruch 1 trägt.

9. Das eln Antigen aufweisende Zelle nach Anspruch 8, wobei die Zelle eine dendritische Zelle ist.

10. Isolierter T-Lymphocyt, welcher selektiv einen Komplex aus einem HLA-Klasse I-Molekül und dem AIK-Peptid der SEQ ID No: 2 bindet.

11. Verwendung des ALK-Peptids der SEQ ID No: 2 oder einer eln Antigen tragenden Zelle, welche dieses Peptid trägt, oder autologer für Komplexe aus einem HLA-A*0201-Molekül und dem oben angegebenen ALK-Peptid spezifischer T-Lymphocyten zur Herstellung einer pharmazeutischen Zusammensetzung für die Behandlung von Tumoren, die ein ALK-Antigen exprimieren.

12. Verwendung nach Anspruch 11, wobei der Tumor ausgewählt wird aus der Gruppe, die aus ALK-positiven Lymphom, Neuroblastom und ALKexprimlerender Neoplasie besteht.

13. Isolierte Nukleinsäure, dle für das HLA-A*0201-bindende ALK-Peptid der SEQ ID No: 2 kodiert.

14. Chimäres Gen, umfassend die isolierte Nukleinsäure nach Anspruch 13 unter Kontrolle eines heterologen Promoters.

15. Expressionsvektor, umfassend das chlmäre Gen nach Anspruch 14.

16. Wirtsvektorsystem zur Herstellung eines Peptids, welches den Expressionsvektor aus Anspruch 15 in einer geeigneten Wirtszelle umfasst.

17. Zell-Linie, umfassend das chimäre Gen aus Anspruch 14.

## Revendications

1. Peptide ALK de liaison à HLA-A*0201 de SEQ ID N° :2.

2. Anticorps monoclonal ou polyclonal, ou un fragment actif de celui-ci, reconnaissant et se liant au peptide ALK de SEQ ID N° :2.

3. Composition pharmaceutique comprenant le peptide ALK selon la revendication 1, en combinaison avec au moins un excipient acceptable sur le plan pharmaceutique.

4. Composition selon la revendication 3, sous la forme d'un vaccin.

5. Procédé destiné à induire ex vivo une réponse cytotoxique contre des cellules tumorales exprimant un antigène ALK, qui comprend la mise en contact des lymphocytes T avec le peptide selon la revendication 1 dans des conditions adaptées à l'activation des lymphocytes T.

6. Procédé selon la revendication 5, dans lequel les lymphocytes T sont directement exposés au peptide en culture.

7. Procédé selon la revendication 5, dans lequel le peptide est préalablement lié à la molécule HLA-A*0201.

8. Cellule présentant un antigène isolé portant le peptide ALK selon la revendication 1.

9. Cellule présentant l'antigène isolé selon la revendication 8, dans laquelle la cellule est une cellule dendritique.

10. Lymphocyte T isolé qui se lie sélectivement à un complexe d'une molécule HLA de classe I et du peptide ALK de SEQ ID N° :2.

11. Utilisation du peptide ALK de SEQ ID N° :2, ou d'une cellule présentant un antigène portant un tel peptide, ou de lymphocytes T autologues spécifiques des complexes d'une molécule HLA-A*0201 et du peptide ALK comme spécifié ci-dessus, pour la préparation d'une composition pharmaceutique destinée à traiter des tumeurs exprimant l'antigène ALK.

12. Utilisation selon la revendication 11, dans laquelle ladite tumeur est choisie dans le groupe consistant en : un lymphome positif pour ALK, un neuroblastome et une néoplasie exprimant ALK.

13. Acide nucléique isolé codant pour le peptide ALK de liaison à HLA-A*0201 de SEQ ID N° :2.

14. Gène chimérique comprenant l'acide nucléique isolé selon la revendication 13, sous le contrôle d'un promoteur hétérologue.

15. Vecteur d'expression comprenant le gène chimérique selon la revendication 14.

16. Système hôte-vecteur destiné à la production d'un peptide, qui comprend le vecteur selon la revendication 15 dans une cellule hôte adaptée.

17. Lignée cellulaire comprenant le gène chimérique selon la revendication 14.


### SEQUENCE LISTING

<110> Gambacorti-Passerini, Carlo
   Passoni, Lorena
<120> Immunogenic ALK Peptides
<130> 045922/241203
<160> 7
<170> FastSEQ for Windows Version 4.0
<210> 1
   <211> 10
   <212> PRT
   <213> Homo sapiens
<400> 1
   Ser Leu Ala Met Leu Asp Leu Leu His Val
   1 5 10
<210> 2
   <211> 10
   <212> PRT
   <213> Homo sapiens
<400> 2
   Gly Val Leu Leu Trp Glu Ile Phe Ser Leu
   1 5 10
<210> 3
   <211> 9
   <212> PRT
   <213> Homo sapiens
<400> 3
   Leu Ala Met Leu Asp Leu Leu His Val
   1 5
<210> 4
   <211> 9
   <212> PRT
   <213> Homo sapiens
<400> 4
   Ala Met Leu Asp Leu Leu His Val Ala
   1 5
<210> 5
   <211> 9
   <212> PRT
   <213> Homo sapiens
<400> 5
   Val Leu Leu Trp Glu Ile Phe Ser Leu
    1               5
<210> 6
   <211> 10
   <212> PRT
   <213> Homo sapiens
<400> 6
   Ala Leu Pro Ile Glu Tyr Gly Pro Leu Val
    1               5                   10
<210> 7
   <211> 9
   <212> PRT
   <213> Homo sapiens
<400> 7
   Ser Leu Thr Ala Asn Met Lys Glu Val
    1               5